**Europäisches Patentamt**

**European Patent Office**

**Office européen des brevets**

(19)

(11) Veröffentlichungsnummer : **0 504 835 A2**

# (12) EUROPÄISCHE PATENTANMELDUNG

(21) Anmeldenummer : **92104677.7**

(51) Int. Cl.$^5$ : **A61N 1/08**

(22) Anmeldetag : **18.03.92**

(30) Priorität : **18.03.91 DE 4108804**

(43) Veröffentlichungstag der Anmeldung :
**23.09.92 Patentblatt 92/39**

(84) Benannte Vertragsstaaten :
**AT CH ES FR GB GR IT LI NL SE**

(71) Anmelder : **Lauerer, Friedrich**
**Karwendelstrasse 12a**
**W-8033 Krailling (DE)**

(72) Erfinder : **Lauerer, Friedrich**
**Karwendelstrasse 12a**
**W-8033 Krailling (DE)**

(54) **Elektromedizinische Schutzschaltung.**

(57) Die Erfindung betrifft eine Schaltung zum Schutze eines Patienten, der zur Untersuchung, zur Therapie oder während einer Operation an elektrischen Geräten (z.B : EKG, EEG, EMG) angeschlossen ist. Die Gefahr vor einem tödlichen Stromstoß ist umso größer, je näher eine Leitung am Herzen liegt (Extremgefahr : Herzkatheter). Sie wächst auch mit der Anzahl der zum Patienten führenden Leitungen und mit der Anzahl der zusätzlich angeschlossenen Meß- und Datenverarbeitungsgeräte (z.B. Computer, Drucker, Bildschirm). Die erfindungsgemäße Schutzschaltung besteht darin, daß zwischen den zum Körper des Patienten führenden Leitungen (1, 2, ...E) spannungsbegrenzende Bauelemente, wie z.B. gegenpolig und parallel geschaltete Gleichrichterdioden (G1, G2, G3 ...Gn) liegen.

EP 0 504 835 A2

Gegenstand der Erfindung ist eine Schutzschaltung, die einen Patienten, der durch Leitungen mit elektromedizinischen Geräten verbunden ist, vor einem gefährlichen Stromfluß schützen soll.

Bei Operationen (hier werden z.B. HF-Chirurgiegeräte angewandt), bei Intensivbehandlung (hier werden dem Patienten lebensnotwendige Soffe zugeführt), beim Messen von Körperaktionsspannungen (z.B. EKG, EEG, EMG) und anderen physikalischen Größen (z.B. Temperatur) sowie bei Therapiemaßnahmen (z.B. Reizstromgerät) werden Leitungen oder/und Geräteteile am bzw. im menschlichen Körper zur Anwendung gebracht, die im Fehlerfalle einen lebensgefährlichen elektrischen Strom durch den Körper, insbesondere durch das Herz zur Folge haben können. Die Gefahr ist nicht nur bei elektrischen Patientenleitungen (z.B. EKG, EEG) gegeben, sondern auch bei sonstigen Leitungen (z.B. Atmung, Bluttransfusion), da die meisten dieser Leitungen mit elektrischen Geräten verbunden sind und die Leitungen selbst elektrisch leitende Flüssigkeiten führen oder leitende Oberflächen haben können.

Zum Schutz gegen diese Gefahren wird im wesentlichen die elektrische Isolierung angewandt, zu der letztlich auch die Trennung von Stromkreisen (z.B. Trenntransformator, Optokoppler) zu zählen ist. Metallene und nicht zum Betriebsstromkreis [gehärende] Geräteteile (z.B. Gehäuse) werden mit einer zusätzlichen Isolierung ("Schutzisolierung") versehen oder mit dem geerdeten Schutzleiter verbunden, wobei allerdings bei Falschanschluß oder Schutzleiter-Unterbrechung mit gleichzeitigem Außenleiterschluß eine vorher nicht gewesene Gefahr erst geschaffen wird. Um auch gegen die zuletzt genannten Gefahren den erforderlichen Schutz gewährleisten zu können, werden die Gerätegehäuse einzeln und zusätzlich über eigene Leitungen am Schutzleiter angeschlossen, d.h. es wird ein redundanter Schutz geschaffen. Dieser zusätzliche Schutzleiteranschluß dient gleichzeitig zum Anschluß aller in der Nähe des Patienten befindlichen fremden leitfähigen Teile. Auf diese Weise wird ein sog. "Potentialausgleich" geschaffen, d.h. diese Teile können keine unterschiedlichen und gefährlichen Spannungen annehmen; allerdings mit dem Nachteil, daß bei gleichzeitiger Berührung von spannungsführenden Teilen eine vorher nicht oder vermindert gewesene Gefahr erst geschaffen bzw. erhöht wurde.

Bei bestimmten medizinischen Behandlungen (z.B. Operationen oder Intensivbehandlung) kann ein Stromausfall für den Patienten lebensgefährlich sein. Aus diesem Grunde darf bei Auftreten eines ersten technischen Fehlers (z.B. Erdschluß) keine Abschaltung erfolgen. Diese Forderung wird dadurch erfüllt, daß die Versorgung über einen Trenntransformator erfolgt und keine der beiden Sekundärleitungen geerdet wird (Isoliertes Netz). Bei Erdschluß erfolgt lediglich eine optische und akustische Meldung, jedoch keine Abschaltung.

Alternativ wird auch der empfindliche Fehlerstrom-Schutzschalter (EFI) angewandt. Er ermöglicht neben der üblichen Anwendung des Schutzleiters eine Abschaltung, wenn der Fehlerstrom einen Wert von meist 30 mA überschreitet. Dieser Schutzschalter bietet daher nicht nur einen Schutz bei direkter Berührung spannungsführender Teile, sondern auch bei spannungsführendem Schutzleiter (z.B. bei Falschanschluß), er bietet einen Schutz vor dem Stromtod überhaupt (bei Erdschluß), weil sein Auslösestrom unterhalb der tödlichen Stromschwelle von ca. 50 mA liegt. Im Berührungsfalle ist die Abschaltung allerdings mit einem spürbaren Stromstoß verbunden, weil bis zum Abschaltende, d.h. in einem Zeitraum von 10 bis 40 ms der volle Unfallstrom, der bis ca. 1 Amp. betragen kann, durch den Körper fließt.

Da der angeführte Schwellenwert 50 mA nur dann gilt, wenn die Stromübergangsstellen relativ weit weg vom Herzen liegen (z.B. an der Außenhaut) und relativ kleine Flächen haben, bietet der EFI-Schutzschalter insbesondere dann keinen Schutz, wenn mindestens eine Stromübergangsstelle am oder im Herzen liegt (z.B. bei Herzkatheteranwendung). Hier können bereits Ströme von ca. 0,08 mA tödlich wirken, wenhalb sicherheitshalber ein Fehlerstrom von max. 0,01 mA zugelassen wird. Ströme in der Größenordnung von 0,08 mA können übrigens auch bei normalem Betrieb auftreten und zwar dann, wenn der Betriebs- oder auch ein Fehlerstrom am Schutzleiter eine Spannung erzeugen kann, oder wenn kapazitive Kopplungen bestehen. Angeblich sind wegen solcher und ähnlicher Ursachen vor zwei Jahrzehnten allein in den USA ca. 1200 Patienten jährlich ums Leben gekommen (Bulletin SEV 1971, Seite 1081).

Auch das oben bereits erwähnte IT-Netz (Trenntransformator mit Isolationsüberwachung) kann in solchen Fällen den notwendigen Schutz nicht bieten, weil eine Fehlermeldung erat dann erfolgt, wenn der Fehlerstrom den Wertebereich von 2,2 bis 4,4 mA überschritten hat. Darüberhinaus ist ein Schutz auch dann nicht gegeben, wenn bei mindestens zwei der angeschlossenen Geräte ein zum Patienten durchgängiger Isolationsfehler vorliegt und beide Trafo-Sekundärleitungen einen Fehlerstrom abgeben.

Die tödliche Stromgefahr ist nicht nur umso größer, je näher eine Leitung am Herzen liegt, sie wächst auch mit der Anzahl der zum Patienten führenden Leitungen (z.B. EKG, EEG, EMG....) und mit der Anzahl der zusätzlich angeschlossenen Meß- und Datenverarbeitungsgeräte wie Rechner mit Bildschirm und Drucker.

Da die zusätzlichen Schutzmaßnahmen den erforderlichen Schutz nicht gewährleisten können, ist dieser letztlich nur dadurch zu realisieren, daß in allen Geräten eine hochwertige Isolierung verwendet wird. Dabei ist auch zu berücksichtigen, daß unvermeidbare kapazitive Kopplungen bereits einen nicht verringerbaren Grundstrom erzeugen. Da der durch das Herz fließende Wechatrom (50 Hz) nicht größer als 0,01 mA sein darf, muß der geforderte Isolationswiderstand mindestens 22 Meg-Ohm betragen.

Zur Realisierung dieser Forderung sind für die betreffenden medizinischen Geräte incl. der Peripherie [geräte] hochspannungsfeste Trenntransformatoren und Optokoppler vorgeschrieben.Wenn auch diese Bauelemente für sich bezüglich ihrer Spannungsfestigkeit den hohen Anforderungen genügen, so konnen andere Umstände den vorhandenen hohen Isolationswiderstand zunichte machen: Im langjährigen Praxisbetrieb kann durch Umwelteinflüsse (z.B. Feuchtigkeit) die Isolation außerhalb der Bauelemente beeinträchtigt werden, z.B. durch Oberflächenleitung von Anschluß zu Anschluß. Derselbe Effekt kann dadurch eintreten, daß aus unvorhergesehener Notwendigkeit unbedacht zusätzliche Peripheriegeräte (z.B. Meßgeräte) hinzugeschaltet werden, welche die geforderte Spannungsfestigkeit nicht besitzen.

Als einzige Schutzmaßnahme gegen die erwähnten Gefahren wird in der Bundesrepublik Deutschland seit 1986 die regelmäßige Überprüfung der elektromedizinischen Geräte vorgeschrieben und durchgeführt. Diese Revisionen sind mit einem erheblichen Personalund Finanzaufwand verbunden (BRD: schätzungsweiae 1,5 Milliarden DM jährlich). Zu bedenken ist außerdem, daß die erzielbare Gefahrenreduzierung verhältnismäßig gering ist; denn ein Isolationsschaden tritt im allgemeinen plötzlich auf. Ein Unfall ist dann nur eine Frage der Zeit, wobei der nächste Prüftermin meist dann keine Rolle mehr spielt.

Vorschläge für effizientere Schutzmaßnahmen sind bekannt geworden. So wird z.B. vorgeschlagen, den Einfluß von kapazitiven Kopplungen durch Verwendung von Gleichstrom auszuschalten (Bio-medizinische Technik Band 34, Ergänzungsband 1989, S. 134). Weiter wird der vermehrte Einsatz von Trenntransformatoren (z.B. für jedes einzelne Gerät) vorgeschlagen (Biomedizinische Technik Band 35, Ergänzungsband 1990, Seite 272). Die Realisierung dieser Vorschläge käme nur für neue Geräte infrage und würde für die im Betrieb befindlichen Geräte keine Lösung bieten. Weiter würde der erste Vorschlag trotz zusätzlichem Aufwand keine gleichwertige Verbesserung bringen. Beim zweiten Vorschlag wäre die Verbesserung zwar besser, aber dennoch ungenügend.

Der vorliegenden Erfindung liegt die Aufgabe zugrunde, mit wenig Aufwand einen Schutz des Patienten zu realisieren, der unabhängig vom Isolationszustand der angeschlossenen Geräte, und ohne daß die fehlerbehaftete Anlage abgeschaltet werden muß, voll wirksam ist.

Die Erfindung löst die gestellte Aufgabe gemäß Anspruch 1 dadurch, daß zwischen allen zum Körper des Patienten führenden Leitungen spannungsbegrenzende Bauelemente liegen. Die folgenden Unteransprüche enthalten vorteilhafte Weiterbildungen der Erfindung.

In den Zeichnungen werden die gegebenen Probleme und die erfin dungsgemäße Lösung an Beispielen dargestellt.

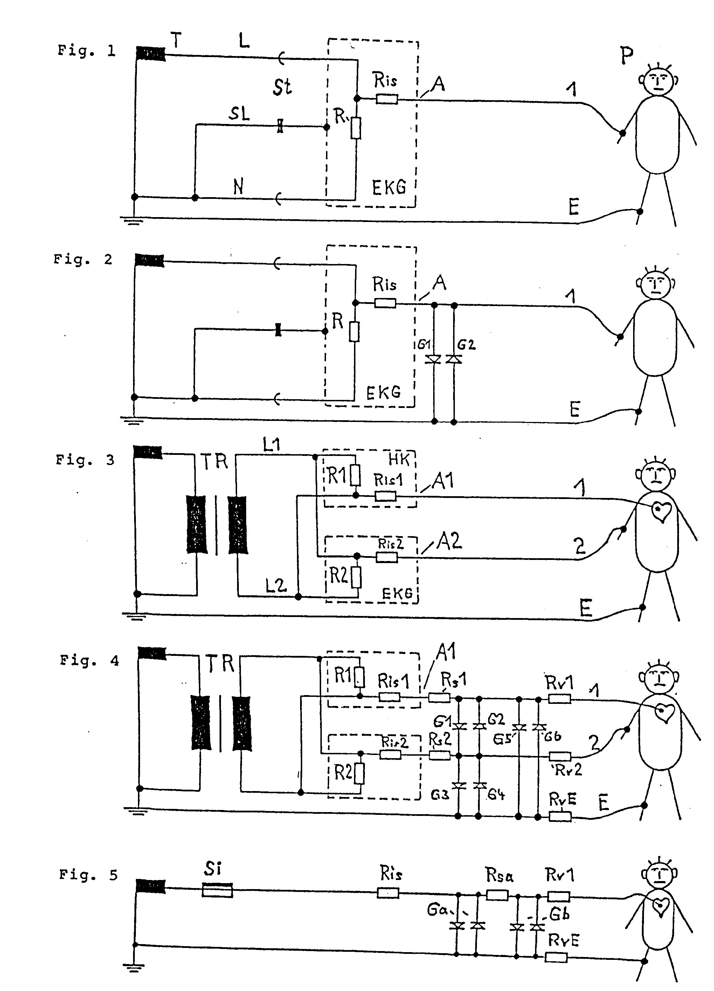

In Fig. 1 ist der Patient P mit der Leitung 1 des EKG-Gerätes verbunden. Der Übersichtlichkeit halber ist neben der Erdleitung E keine weitere Ableitung eingezeichnet. Das Gerät wird über die Schutzkontaktsteckdose St direkt vom allgemeinen Netztransformator T über die Leitungen L und N versorgt. R bedeutet den Verbraucherwiderstand des Gerätes EKG und A ist die Auslaßstelle für die Patientenleitung 1. Das Gerätegehäuse selbst ist am Schutzleiter SL angeschlossen.

Tritt im Gerät vom Außenleiter L ein Schluß zur Geräteauslaßstelle A auf, d.h. ist der dazwischenliegende Isolationswiderstand Ris unzulässig klein (z.B. 2 000 Ohm), dann besteht für den Patienten Lebensgefahr (88 mA Unfallstrom bei 220 Volt und 500 Ohm Körperwiderstand).

In Fig. 2 ist dieselbe Situation wie bei Fig. 1 dargestellt, nur daß hier zusätzlich die erfindungsgemäße Schutzschaltung (G1, G2) angewandt wird. Bei einem gefährlichen, d.h. geringen Isolatiönswiderstand Ris, z.B. 2 000 Ohm, fällt am Spannungsbegrenzer, d.h. an den gegenpolig parallelgeschalteten Gleichrichtern G1 und G2 lediglich eine Spannung von 0,5 Volt ab mit der Folge, daß durch den Körper des Patienten höchstens 1 mA fließen. Der Strom ist völlig ungefährlich, wenn die Stromübergangsstellen, wie hier, an der Körper-Außenfläche bzw. genügend weit vom Herzen entfernt liegen.

Da die vom menschlichen Körper nach außen abgegebenen und zu, messenden Körperaktionsspannungen (z.B EKG, EEG, EMG) kaum über 2 mV (in Ausnahmefällen bei EKG-Messungen in Herznähe bis 7 mV) betragen, stört der Spannungsbegrenzer, bei dem die Begrenzungsfähigkeit im günstigsten Fallerst bei 80 mV beginnt, die Messungen nicht, da er bei so kleinen Anlegespannungen einen Eigenwiderstand von mehreren 100 Meg-Ohm hat. Die zu den Stromquellen im menschlichen Körper (z.B. Herz, Muskel) parallel geschalteten Körperwiderstände liegen um mehrere Größenordnungen tiefer. Auch die in Fig. 4 angegebenen Strombegrenzungswiderstände (Rs und Rv) beeinflussen die Meßergebnisse kaum, wenn sie z.B. 10 K-Ohm nicht überschreiten.

In Fig. 3 ist zusätzlich zum EKG ein Herzkathetergerät HK angeschlossen. Solange bei Erdung des Patienten der Isolationswiderstand des Gerätes Ris den Wert 22 Meg-Ohm nicht unterschreitet, überschreitet der durch den Patienten fließende Ableitstrom den Wert von 0,01 mA nicht, was Gefahrenfreiheit bedeutet.

Angenommen der Isolationswiderstand im Herzkathetergerät HK beträgt Ris1 = 2 Meg-Ohm, dann fließt von dem im Herzen liegenden Katheter ein Strom von 0,11 mA durch den Körper zur Erde E, was mit einer Lebensgefahr verbunden ist. Die Gefahr wäre bei Weglassung der Erdleitung E nicht wesentlich geringer, weil ein zuzätzlicher Isolationsfehler in einem anderen Gerät (z.B. Ris2), oder/und die Körperberührung durch eine zweite Person (z.B. Arzt, der selbst keinen Strom spüren muß!), oder/und kapazitive Kopplungen zur Erde eine unvermeidbare relativ niederohmige Verbindung zur Erde herstellen.

In Fig. 4 ist dieselbe Situation wie bei Fig. 3 dargestellt, nur daß hier zusätzlich die erfindungsgemäße Schutzschaltung angewandt wird. Bei einem für Herzkatheteranwendung gefährlichen Isolationswiderstand z.B. Ris = 2 Meg-Ohm fällt am Spannungsbegrenzer G5 und G6 zwar noch eine Spannung von z.B. 0,4 Volt ab, doch die Widerstände Rvl und RvE (z.B. mit je 10 K-Ohm) begrenzen den durch das Herz fließenden Strom auf einen ungefährlichen Wert, hier auf 0,02 mA.

Wie aus der Schaltung ersichtlich, ist der Patient nicht nur bei einen zweifachem Leiterschluß geschützt, sondern auch bei direkter Berührung eines spannungsführenden Leiters oder direkter Berührung der Erde.

In Fig. 5 ist die Schutzschaltung in verbesserter Ausführung dargestellt. Durch Kaskadenschaltung, hier Ga und Gb, kann die Spannungsbegrenzung in Verbindung mit Ras auf einen noch tieferen Wert herabgesetzt werden. Außerdem kann durch geeignete Wahl des ersten Spannungsbegrenzers Ga such ein niederohmige Isolationswiderstand (z.B. Kurzschluß) zu keinem Schaden führen, weil in diesem Falle die vorgeschaltete Sicherung Si die Stromzufuhr unterbricht.

## Patentansprüche

1. Elektromedizinische Schutzschaltung zur Verhinderung von gefährlichen Strömen im menschlichen Körper durch Leitungskontakt, dadurch gekennzeichnet, daß zwischen allen zum Körper führenden Leitungen (1, 2, ...E) spannungsbegrenzende Bauelemente (G1, G2, G3 ...Gn) liegen.

2. Elektromedizinische Schutzschaltung nach Anspruch 1, dadurch gekennzeichnet, daß als spannungsbegrenzende Bauelemente Halbleiter (z. B. Gleichrichter, Schottky-Dioden oder Transistoren) verwendet werden und jeweils zwei Bauelemente gegenpolig parallel geschaltet sind.

3. Elektromedizinische Schutzschaltung nach Anspruch 1 oder 2, dadurch gekennzeichnet, daß der Patient an einer Stelle, z.B. an einem Bein, mit dem Erdleiter (E) verbunden ist.

4. Elektromedizinische Schutzschaltung nach einem der vorhergehenden Ansprüche, dadurch gekennzeichnet, daß bei jeder Zuleitung zwischen den Geräteauslaßstellen (A, A1, A2 ...An) und den Spannungsbegrenzern (G1, G2, G3, ....Gn) ein Strombegrenzungswiderstand (Rs1, Rs2, ....Rsn) liegt.

5. Elektromedizinische Schutzschaltung nach einem der vorhergehenden Ansprüche, dadurch gekennzeichnet, daß zwischen den Spannungsbegrenzern (G1, G2, G3 ... Gn) und dem Körper des Patienten je ein strombegrenzender Vorwiderstand (Rv1, Rv2 ... RvE) liegt.

6. Elektromedizinische Schutzschaltung nach einem der vorhergehenden Ansprüche, dadurch gekennzeichnet, daß eine oder mehrere Spannungsbegrenzer (Ga, Gb, ...Gn) parallel geschaltet werden und dazwischen jeweils ein zusätzlicher Strombegrenzungswiderstand (Rsa, Rsb....Rsn) liegt.

7. Elektromedizinische Schutzschaltung nach einem der vorhergehenden Ansprüche, dadurch gekennzeichnet, daß die Spannungsbegrenzer bei Gas- oder Flüssigkeitsleitungen jeweils an der Rohr-Innenwand angeschlossen sind, die in einer begrenzten Länge mit einer elektrisch leitenden Schicht versehen ist.

8. Elektromedizinische Schutzschaltung nach einem der vorhergehenden Ansprüche, dadurch gekennzeichnet, daß die zum Patienten führenden Leitungen über einen gemeinsamen Verteilerkasten führen, in dem die Schutzschaltung untergebracht ist und an dem sowohl die einzelnen medizinischen Geräte, wie auch die zum Patienten führenden Leitungen mittels Steckvorrichtungen angeschlossen sind.

9. Elektromedizinische Schutzschaltung nach einem der vorhergehenden Ansprüche, dadurch gekennzeichnet, daß die medizinischen Geräte über einen Trenntransformator (TR) gespeist werden und die Sekundärleitungen an einem Isolationsüberwachungsgerät angeschlossen sind.

10. Elektromedizinische Schutzschaltung nach einem der vorhergehenden Ansprüche, dadurch gekennzeichnet, daß Trenntransformator (TR), Isolationsüberwachung und der Verteilerkasten nach Anspruch 8 in einem gemeinsamen Gehäuse untergebracht sind, an dem sowohl die einzelnen Geräte (G1, G2 ..,Gn), wie auch die zum Patienten führenden Leitungen (1, 2 ...E) mittels Steckvorrichtungen angeschlossen sind.

Fig. 1

Fig. 2

Fig. 3

Fig. 4

Fig. 5